(19) Europäisches Patentamt
European Patent Office
Office européen des brevets

(11) Publication number: 0 132 764 B1

(12) EUROPEAN PATENT SPECIFICATION

(45) Date of publication of patent specification: 02.01.91

(51) Int. Cl.$^5$: C 07 C 211/32

(21) Application number: 84108424.7

(22) Date of filing: 17.07.84

(60) Divisional application 89114293.7 filed on 02/08/89.

# (54) Derivatives of dihydrodibenzocycloheptylidene-ethylamine.

(30) Priority: 02.08.83 ES 524680

(43) Date of publication of application: 13.02.85 Bulletin 85/07

(45) Publication of the grant of the patent: 02.01.91 Bulletin 91/01

(84) Designated Contracting States: AT BE CH DE FR GB IT LI NL SE

(56) References cited:
EP-A-0 056 616
CH-A- 383 997
US-A-4 308 387
US-A-4 381 305

THE JOURNAL OF ORGANIC CHEMISTRY, vol. 27, January-April 1962, Easton, Pennsylvania; S.O. WINTHROP et al. "New Psychotropic Agents" pages 230-240

The file contains technical information submitted after the application was filed and not included in this specification

(73) Proprietor: SOCIEDAD ESPANOLA DE ESPECIALIDADES FARMACO-TERAPEUTICAS, S.A.
173, Avda. San Antonio Ma Claret
Barcelona-26 (ES)

(72) Inventor: Cirera, Xavier D.
271, Avenida Rep. Argentina
Barcelona 23 (ES)
Inventor: Andreoli, Romeo R.
6, Calle Roca i Batlle
Barcelona 23 (ES)
Inventor: Lloveras, Pedro P.
94, Calle Viveret
Tarrasa, (Barcelona) (ES)
Inventor: Bruseghini, Léonida
5, Calle Caponata
Barcelona 34 (ES)
Inventor: Irrure, José P.
184, Calle Mayor de Sarriá
Barcelona 17 (ES)

(74) Representative: Sandmair, Kurt, Dr. et al
Patentanwälte Schwabe, Sandmair, Marx
Stuntzstrasse 16
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 132 764 B1

Courier Press, Leamington Spa, England.

**Description**

The present invention concerns new compounds derived from dihydrodibenzocycloheptylidene-ethylamine which possess antiulcer activity, a process for preparing these compounds and pharmaceutical compositions containing them.

In J. Org. Chem. 27, 230—240 (1962) an antidepressive activity of dibenzo[a,d]-1,4-cycloheptadiene derivatives is disclosed.

US—A—4381305 discloses a vascular antispasmodic and antiallergic activity of certain 1,2 ethylenediamine derivatives.

US—A—4308387 discloses certain diphenylbutyl-piperazine-carboxamide derivatives which possess antipsychotic properties.

CH—A—383997 discloses a method for preparing certain new ethylidene derivatives of fluorene or thioxanthene having antihistaminic activity.

EP—A—0056616 discloses certain cycloheptene derivatives having antihistaminic activity.

The compounds according to the present invention have the following structural formula (I)

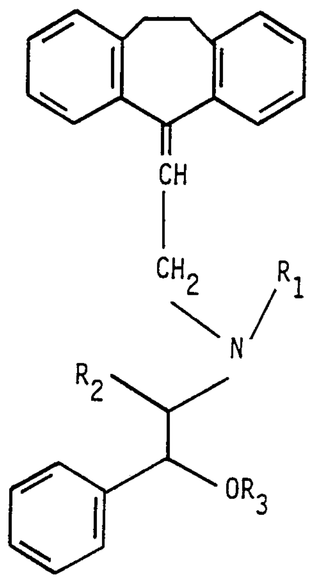

(I)

in which $R_1$, $R_2$ and $R_3$ are defined as follows:

$R_1$: H, $CH_3$

$R_2$: H, $CH_3$,

$R_3$: H, $C_1$—$C_4$-alkyl, CO-($C_1$—$C_4$-alkyl).

The invention also refers to the pharmaceutically acceptable salts of the compounds of formula (I), to the process for the obtention of said compounds and their derivatives.

Said compounds possess a marked antiulcer activity.

The process for the obtention of the compounds defined by the general formula (I) is characterized by the reaction of an amine of general formula (II)

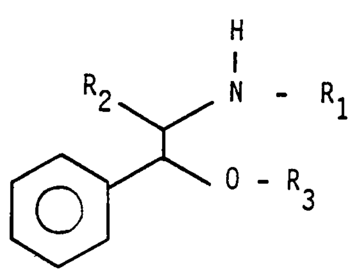

(II)

where $R_1$, $R_2$ and $R_3$ possess the same significance as in the general formula (I) with a halogen derivative which has the general formula (III)

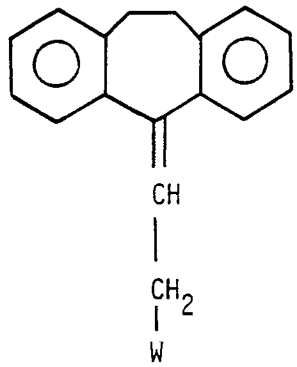

(III)

wherein W is chlorine or bromine.

The reaction is carried out in an inert solvent in presence of a hydrogen halide binder, which may be an inorganic or organic base, or an excess of the original amine.

The compounds of general formula (I) may also be prepared by reacting the amines of general formula (II) with an aldehyde of formula (IV)

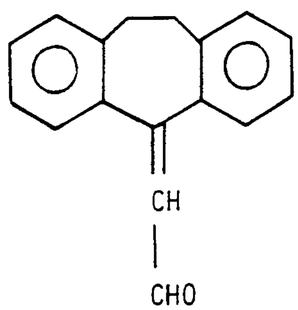

(IV)

in the presence of a reducing agent such as sodium borohydride or catalytic hydrogenation.

An alternative form of isolation of the compounds (I) may be by formation of salts with mineral acids such as hydrochloric, sulphuric or nitric acid, or with organic acids such as, for example, oxalic, salycylic, citric, maleic or fumaric acid. The utilization of hydrochloric or maleic acid is preferable, due to their pharmacological properties.

The compounds of formula (I) are designed briefly by the references as follows: the alphabetic expression "WAS- . . ." followed by a specific number which is exclusive for each formula.

Examples of Chemical Synthesis

Examples 1 and 2 show the chemical synthesis of the compounds according to the present invention:

Example 1

Obtention of N-2-(10,11-dihydrodibenzo-(a,d)cyclohept-5-ylidene)-ethyl-ephedrine

This compound is briefly designated as WAS-4304.

A mixture of 5.10 g (20 mmol) of 2-chloro-1-(10,11-dihydrodibenzo-(a,d)-cyclohept-5-yliden)-ethane and 6.60 g (40 mmol) of ephedrine base were refluxed in 100 ml of acetonitril for 4 hr. After cooling, the solvent was eliminated and the residue taken up in chloroform and washed with 10% hydrochloric acid. The organic extract was dried over anhydrous sodium sulphate, the solvent eliminated and the residue recrystalized from acetonitril. 5.03 g of N-2-(10,11-dihydrodibenzo-(a,d)-cyclohept-5-ylidene)-ethylephedrine were obtained. Yield: 62%.

Analytical data:

Melting point: 231—232°C

IR: 3370, 2620, 2510, 1600, 1485, 775, 760, 745, 705.

NMR: 7,4/sc(13H); 6,4/t(1H); 6,0/t(1H); 5,5/sc(1H), 2,5—4,2/sc(8H).

In Table 1, examples 2 to 4 further illustrate the present invention. In each case, the specific chemical structure and the analytical data which correspond to each of said compounds are shown. The method of synthesis corresponds to that of example 1.

In said Examples 1 to 4 the abbreviation IR refers to the infrared spectrum and the abbreviation NMR signifies the proton nuclear magnetic resonance spectrum. The given NMR-data indicate in the first place,

the position of the signal in parts per million (scale δ); the form or multiplicity of the signal is indicated by the following abbreviations:

S = singlet
d = doublet
t = triplet
q = quadruplet
sc = complex signal
sa = broad signal

The number of protons corresponding to each signal, obtained by electronic integration, is indicated between brackets.

TABLE 1

| No. of Example | Compound denomination | $R_1$ | $R_2$ | $R_3$ |
|---|---|---|---|---|
| 2 | WAS-4389 | $CH_3$ | $CH_3$ | 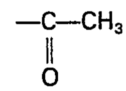 |
| 3 | WAS-4390 | $CH_3$ | $CH_3$ | 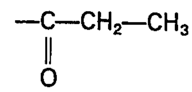 |
| 4 | WAS-4391 | $CH_3$ | $CH_3$ | $—CH_3$ |

TABLE 1 cont.

| No. of Examples | melting point [°C] | NMR |
|---|---|---|
| 2 | 203—204 (hydrochloride) | 7,4/sc(13); 6/4/t(1); 6,0/t(1); 5,5/sc(1); 2,5—4,2/sc(8); 2,8/s(3); 2,1/s(3); 1,8/sc(3). |
| 3 | 197—198 (hydrochloride) | 7,4/sc(13) 6,3/t(1); 5,9/t(1) 5,5/sc(1); 2,5—4,2/sc(10); 2,9/s(3); 1,9/sc(3); 1,7/t(3) |
| 4 | 189—190 (hydrochloride) | 7,4/sc(13); 6,4/t(1); 6,0/t(1); 5,5/sc(1); 3,5/s(3); 2,5—4,2/sc(7); 2,8/s(3); 1,8/sc(3). |

PHARMACOLOGICAL PROPERTIES

The compounds of the present invention possess antiulcer activity. In the following, the method for evaluation of the above-mentioned pharmacological activity is described, together with the results obtained.

Antiulcer Activity

In order to evaluate this activity, the results of which are set out in the following Table 2, the method followed was that of antagonism to indomethacine-induced ulcers in the rat according to that described by

K. P. Bhargava, M. B. Grupta and K. K. Tangri (Eur. J. Pharm. *22*, 191—195 (1973)). The $ED_{50}$ for antiulcer activity has been calculated for the majority of the product which showed greatest activity and the action of the remaining products has been expressed using the system of crosses for comparison with the standard drug, employing the symbols and criteria as given in the following. The standard drugs used in this experiment were ranitidine and cimetidine.

The results have been symbolically expressed by crosses, the number of which is proportional to the intensity of effect, the maximum value of four crosses representing an intensity of action comparable to the standard drug used. The significance of the symbols is as follows:

++++ Very intense activity
+++ Considerable activity
++ Moderate activity
+ Low activity
0 Null activity

TABLE 2

| Compound | Example No. | Antiulcer Activity | |
|---|---|---|---|
| | | Evaluation | $ED_{50}$ mg/kg p.o. |
| WAS-4304 | 1 | ++++ | 0.069 |
| Ranitidine | | ++++ | 8.2 |
| Cimetidine | | ++++ | 28.2 |

Toxicity

The indicative $LD_{50}$ (mg/kg i.p.) WAS-4304 for is 115.

The said compound was administered by the intraperitoneal route to Swiss mice, observing the toxic effects, and calculating the mortality rate and the $LD_{50}$ 7 days post-administration.

The compounds described in the present invention have the following therapeutical applications:

Gastric and duodenal ulcer, Zollinger-Ellison syndrome, digestive hemmorrhage due to lesions of the gastric mucosal layer. Recurrent and post-anastomotic surgical ulcers. Peptic oesophagitis.

The compounds of the present invention can be administered under all the pharmaceutical forms compatible with their pharmacotechnical and therapeutic properties, at an adequate dosage.

**Claims**

1. Derivatives of dihydrodibenzocycloheptylidene-ethylamine of the general formula (I)

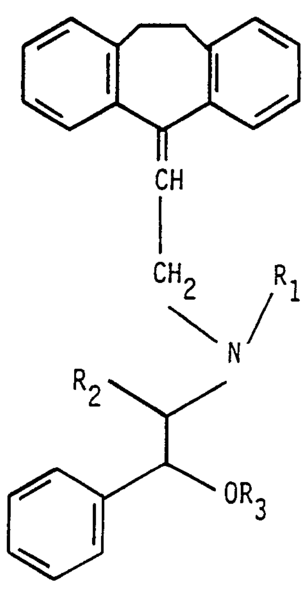

(I)

in which $R_1$, $R_2$ and $R_3$ are defined as follows:

$R_1$: H, $CH_3$;
$R_2$: H, $CH_3$;
$R_3$: H, $C_1$—$C_4$-alkyl, CO-($C_1$—$C_4$-alkyl)

and pharmaceutically acceptable salts thereof.

2. A process for preparing the compounds of claim 1, characterized by the reaction of an amine of the general formula (III)

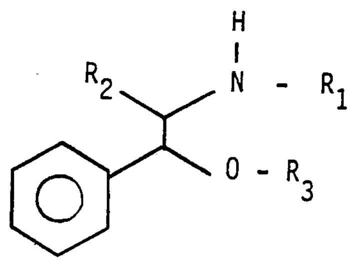

(II)

wherein $R_1$, $R_2$ and $R_3$ have the same significance as in the general formula of claim 1 with a halogen derivative of the general formula (III)

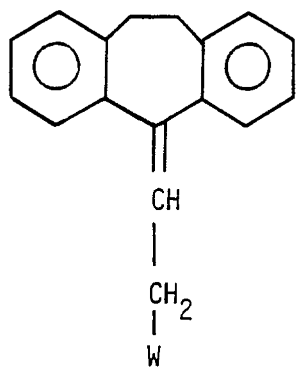

(III)

wherein W is a chlorine or bromine.

3. A pharmaceutical composition comprising as active ingredient a compound of claim 1, together with a pharmaceutically acceptable carrier therefor.

4. A compound according to claim 1 for use in antiulcer therapy.

5. A compound according to claim 1 wherein $R_1$ represents $CH_3$, $R_2$ represents $CH_3$ and $R_3$ represents H.

**Patentansprüche**

1. Dihydrodibenzocycloheptyliden-ethylamin-Derivate der allgemeinen Formel (I):

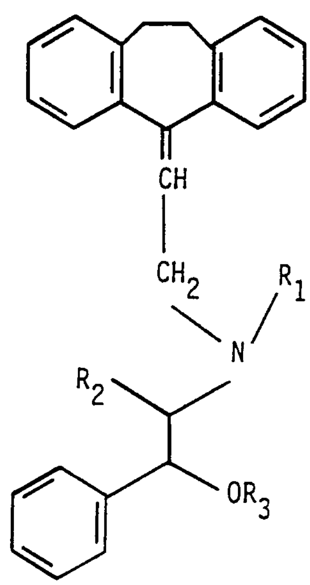

(I)

in der $R_1$, $R_2$ und $R_3$ die folgenden Bedeutungen aufweisen:
$R_1$: H, $CH_3$
$R_2$: H, $CH_3$
$R_3$: H, $C_1$—$C_4$-Alkyl, CO-($C_1$—$C_4$-alkyl)
und deren pharmazeutisch annehmbare Salze.

2. Verfahren zur Herstellung der Verbindungen gemäß Anspruch 1, dadurch gekennzeichnet, daß man ein Amin der allgemeinen Formel (II):

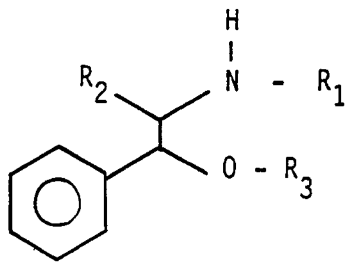

(II)

in der $R_1$, $R_2$ und $R_3$ dieselbe Bedeutung wie in der allgemeinen Formel in Anspruch 1 aufweisen, mit einem Halogenderivat der allgemeinen Formel (III):

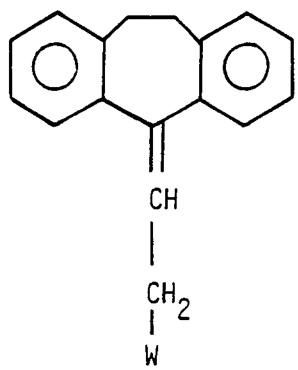

(III)

in der W Chlor oder Brom bedeutet, umsetzt.

3. Pharmazeutische Zusammensetzung, enthaltend als Wirkstoff eine Verbindung gemäß Anspruch 1 zusammen mit einem pharmazeutisch annehmbaren Träger.

4. Verbindung nach Anspruch 1 zur Verwendung in der Antiulcus-Therapie.

5. Verbindung nach Anspruch 1, in der $R_1$ $CH_3$, $R_2$ $CH_3$ und $R_3$ H bedeutet.

**Revendications**

1. Dérivés d'éthylamine de dihydrodibenzocycloheptylidène de la formule générale (I) suivante:

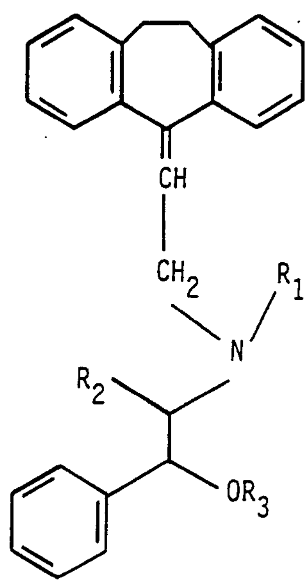

(I)

dans laquelle $R_1$, $R_2$ et $R_3$ représentent
$R_1$: H, $CH_3$
$R_2$: H, $CH_3$
$R_3$: H, $C_1$—$C_4$-alkyl, CO-($C_1$—$C_4$-alkyl)
ainsi que leurs sels, acceptables dans les applications pharmaceutiques.

2. Procédé de fabrication de composés suivant la revendication 1, caractérisé en ce qu'on fait réagir une amine de la formule générale (II)

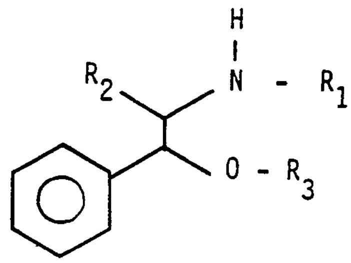

(II)

dans laquelle $R_1$, $R_2$ et $R_3$ représentent les mêmes radicaux que dans la formule générale (I) de la revendication 1 avec un dérivé d'halogène de la formule générale (III),

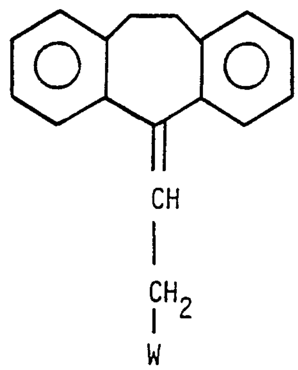

(III)

dans laquelle W représente du chlore ou du brome.

3. Composition pharmaceutique comprenant comme constituant actif un composé suivant la revendication 1, ensemble avec un support acceptable dans les applications pharmaceutiques.

4. Composé suivant la revendication 1 destiné aux thérapies anti-ulcéreuses.

5. Composé suivant la revendication 1 dans lequel $R_1$ représente $CH_3$, $R_2$ représente $CH_3$ et $R_3$ représente H.